# EUROPÄISCHE PATENTSCHRIFT

(11) **EP 0 473 023 B1**
(45) Veröffentlichungstag und Bekanntmachung des Hinweises auf die Patenterteilung: **07.12.1994**
(21) Anmeldenummer: 91113729.7
(22) Anmeldetag: 16.08.1991
(51) Int. Cl.: C07C 45/45, C07C 49/76

(54) **Verfahren zur Herstellung von Acylbenzolen**
Process for the production of acyl benzenes
Procédé pour la préparation d'acyl benzènes

(30) Priorität: 29.08.1990 DE 4027276
(43) Veröffentlichungstag der Anmeldung: 04.03.1992
(73) Patentinhaber: BAYER AG, 51368 Leverkusen (DE)
(72) Erfinder: Waldmann, Helmut, Dr., W-5090 Leverkusen (DE); Hajek, Manfred, Dr., W-5090 Leverkusen (DE); Immel, Otto, Dr., W-4150 Krefeld (DE); Puppe, Lothar, Dr., W-5093 Burscheid (DE); Braden, Rudolf, Dr., W-5068 Odenthal (DE)

(56) Entgegenhaltungen:
- EP-A- 0 239 383
- EP-A- 0 279 322
- DE-A- 2 616 583
- FR-A- 2 592 039

## Beschreibung

Die Erfindung betrifft ein Verfahren zur Herstellung von Acylbenzolen durch Umsetzung von Benzol mit Carbonsäuren an sauren Katalysatoren.

Acylbenzole sind vielseitig einsetzbare Zwischenprodukte für die Synthese von Wirkstoffen auf dem Gebiet der Arzneimittel und des Pflanzenschutzes. Benzophenon dient darüber hinaus in größeren Mengen als Geruchsfixateur für Parfüme und Seifen.

Die Herstellung von Acylbenzolen erfolgt im allgemeinen durch Acylierung von Benzol mit Acylchloriden unter Verwendung von Friedel-Crafts-Katalysatoren, wie Aluminiumchlorid, Zinkchlorid, Eisenchlorid und ähnlichen dem Fachmann bekannten Metallhalogeniden. Diese Friedel-Crafts-Katalysatoren werden im allgemeinen in stöchiometrischen Mengen benötigt. Bei der Aufarbeitung solcher Acylierungsgemische werden die Friedel-Crafts-Katalysatoren hydrolytisch zerstört und ergeben größere Mengen an Salzsäure im Abgas oder im Abwasser. Diese zu entsorgende Salzsäure stammt sowohl aus dem Katalysator als auch aus dem zur Acylierung eingesetzten Acylchlorid. Neben dieser Entsorgung als beträchtlichem Umweltproblem muß auch das Korrosionsproblem, das durch die Salzsäure hervorgerufen wird, gelöst werden. Es besteht daher die Aufgabe, ein Verfahren zu finden, das sowohl umweltfreundlich als auch kostengünstig bezüglich der aufgezeigten Nachteile ist.

Ein erster Ansatz zur Lösung dieser Aufgabe ist in DE-OS 26 16 583 beschrieben, in der vorgeschlagen wird, Arylketone durch Umsetzung von nicht hydroxylierten aromatischen Verbindungen mit einer Carbonsäure oder einem Carbonsäureanhydrid in der Dampfphase bei 250 bis 500°C in Gegenwart eines sauren Kieselsäure/Tonerde-Katalysators mit einer Oberfläche von mindestens 50 m³ /g herzustellen. Bei diesem Verfahren unter Verwendung von amorphen oder kristallinen Kieselsäure/Tonerde-Katalysatoren entstehen jedoch unerwünschte Nebenprodukte sehr unterschiedlicher Struktur, beispielsweise bei der Umsetzung von Benzol mit Benzoesäure neben dem gewünschten Benzophenon noch Biphenyl, Diphenylmethan und alkylierte Benzole.

Ein besonders gravierender Nachteil dieses Verfahrens ist die geringe Lebensdauer der Katalysatoren; so sinkt die Menge an dem gewünschten Benzophenon im Vergleich der Beispiele 2 und 3 von DE-OS 26 16 583 beträchtlich ab, wenn die Laufzeit der Umsetzung auf 24 h erhöht wird.

In EP-A-279 322 wird die Herstellung von Arylketonen, wie substituierten Benzophenonen, in der Gasphase an Zeolithen aus der Gruppe von Mordenit, ZSM-5 oder Fanjasit in der H⁺-Form beschrieben; bevorzugte Acylkomponenten sind aromatische Säurechloride oder -anhydride.

Ebenfalls in der Gasphase und an Molekularsieben des Pentasil-Typs arbeitet das Verfahren von EP-A-239 383, worin jedoch andere Ketone hergestellt werden, nämlich solche mit einem substituierten Arylteil und einem niederen Alkylteil, wozu substituierte Benzole mit niederen aliphatischen Carbonsäuren umgesetzt werden.

Es wurde nun gefunden, daß beim Einsatz ausgewählter definierter Zeolithe verbesserte Selektivitäten und wesentlich erhöhte Standzeiten erreicht werden können. Solche ausgewählten Katalysatoren sind die weiter unten näher beschriebenen Zeolithe vom Pentasil-Typ.

Es wurde ein Verfahren zur Herstellung von Acylbenzolen der Formel 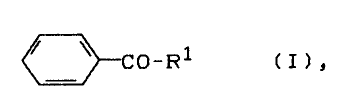
worin
- R¹: für -C₆H₄-R², worin R² Wasserstoff, Methyl, Ethyl, Isopropyl oder geradkettiges C₃-C₁₄-Alkyl bedeutet, steht,
durch Umsetzung von Benzol mit Carbonsäuren an sauren Katalysatoren gefunden, das dadurch gekennzeichnet ist, daß man eine Carbonsäure der Formel

HOOC-R¹ (II),

worin R¹ die obige Bedeutung hat,
in freier Form mit Benzol im Molverhältnis Benzol:Carbonsäure = 1-50:1 bei einer Temperatur von 200-400°C in der Gasphase umsetzt und als sauren Katalysator einen Zeolith vom Pentasil-Typ mit einem SiO₂/AlO₂-Verhältnis von 15-500:1 benutzt, wobei als austauschbare Kationen solche von Mg oder Ca vorliegen.

Als geradkettiges C₃-C₁₄-Alkyl sei beispielsweise n-Propyl, n-Butyl, n-Hexyl, n-Octyl, n-Decyl, n-Dodecyl oder n-Tetradecyl genannt.

In besonderer Weise für das erfindungsgemäße Verfahren sind Carbonsäuren der Formel (I) geeignet, in denen R¹ für -C₆H₄-R², worin R² Wasserstoff, Methyl oder Ethyl bedeutet, steht. In besonders bevorzugter Weise ist Benzoesäure geeignet. Insbesondere sei die Herstellung von Benzophenon aus Benzol und Benzoesäure nach dem erfindungsgemäßen Verfahren genannt.

Benzol wird im mindestens äquimolaren Verhältnis zur Carbonsäure eingesetzt. In bevorzugter Weise verwendet man jedoch einen Überschuß an Benzol, beispielsweise in einem Molverhältnis Benzol:Carbonsäure = 5-30, besonders bevorzugt = 10-20.

Das erfindungsgemäße Verfahren ist besonders durch die Benutzung eines Zeolithes vom Pentasil-Typ als Katalysator gekennzeichnet.

Zeolithe sind durch die allgemeine Formel (III) charakterisiert:

M¹ₘ[mM²O₂·nSiO₂]·qH₂O (III).

Hierin bedeuten
- M¹: ein Äquivalent eines austauschbaren Kations, dessen Anzahl m dem Anteil von M² entspricht;
- M²: ein dreiwertiges Element, welches gemeinsam mit dem Si das oxydische Gerüst des Zeoliths bildet;
- n/m: das SiO₂/M²O₂-Verhältnis;
- q: die Menge des adsorbierten Wassers.

Zeolithe sind von ihrer Grundstruktur her kristalline Alumosilikate, die aus einem Netzwerk von SiO₄- bzw. M²O₄-Tetraedern aufgebaut sind. Die einzelnen Tetraeder sind mit Sauerstoffbrücken über die Ecken der Tetraeder untereinander verknüpft und bilden ein räumliches Netzwerk, das gleichmäßig von Kanälen und Hohlräumen durchzogen ist Die einzelnen Zeolithstrukturen unterscheiden sich durch die Anordnung und Größe der Kanäle und Hohlräume sowie durch ihre Zusammensetzung. Als Ausgleich für die negative Ladung des Gitters, die durch den Anteil an M² zustande kommt, sind austauschbare Kationen eingelagert. Die adsorbierte Wasserphase q H₂O ist reversibel entfernbar, ohne daß das Gerüst seine Struktur verliert.

M² ist erfindungsgemäß Aluminium.

Eine ausführliche Darstellung von Zeolithen ist beispielsweise in der Monographie von D.W. Breck "Zeolite Molecular Sieves, Structure, Chemistry, and Use", J. Wiley & Sons, New York, 1974, gegeben. Eine weitere Darstellung, insbesondere der an SiO₂-reicheren Zeolithe, die für die katalytische Anwendung besonders interessant sind, findet sich in der Monographie von P.A. Jacobs und J.A. Martens "Synthesis of High-Silica Aluminosilicate Zeolites", Studies in Surface Science and Catalysis Ed. B. Delmon und J.T. Yates, Elsevier, Amsterdam-Oxford-New York-Tokyo 1987.

Die erfindungsgemäß einsetzbaren Zeolithe gehören zum Pentasil-Typ; sie sind mittelporig mit Porenweiten von etwa 5 Å.

In bevorzugter Weise werden unter den Pentasil-Typen die Zeolith-Strukturen ZSM 5, ZSM 11, ZSM 8, ZSM 5/ZSM 11 Intermediates, Zeta 1, Zeta 2, ZBM 10, Ultrasil, Ultrazel, TZ-01, NU-4, NU-5, AZ 1 oder ein Gemisch mehrerer von ihnen eingesetzt. In besonders bevorzugter Weise werden die Zeolith-Strukturen ZSM 5, ZSM 11, ZSM 8 und ZSM 5/ZSM 11 Intermediates eingesetzt.

Als austauschbare Kationen M¹ können die genannten Zeolithe solche des Mg oder Ca enthalten.

Während das in Formel (III) definierte SiO₂/M²O₂-Verhältnis n/m bei Zeolithen ganz allgemein Werte von 1 bis 3.000 oder größer annehmen kann, werden für das erfindungsgemäße Verfahren Zeolithe vom Pentasil-Typ eingesetzt, bei denen n/m Werte von 15 - 500 annimmt. In bevorzugter Weise werden n/m-Werte von 30 - 300, in ganz besonders bevorzugter Weise solche von 50 - 150 benutzt. Ersetzt man das Verhältnis SiO₂/M²O₂ durch den Ausdruck SiO₂/M²₂O₃, so verdoppeln sich die Werte von n/m.

Die Einsatzprodukte werden gasförmig an den Katalysator herangebracht. Hierzu wählt man eine Temperatur im Bereich von 200 bis 400°C, bevorzugt 250 bis 300°C. Auch bei Temperaturen darunter oder darüber findet die beschriebene Umsetzung noch statt, jedoch muß mit verringerter Reaktionsgeschwindigkeit bzw. mit dem Auftreten von Nebenprodukten gerechnet werden.

Der Reaktionsdruck ist für das erfindungsgemäße Verfahren von untergeordneter Bedeutung. So kann ein Unterdruck insbesondere angewendet werden, um bei hoch siedenden Einsatzstoffen die Umsetzungsweise in der Gasphase zu ermöglichen; andererseits ermöglicht ein Druck oberhalb des atmosphärischen Druckes eine höhere Raumausbeute. Wegen der verfahrenstechnisch einfacheren Durchführung wird man jedoch möglichst in der Nähe des atmosphärischen Druckes arbeiten.

Der Zeolith-Katalysator kann im Festbett angeordnet sein, er kann jedoch auch in Form eines Fließ- oder Wirbelbettes eingesetzt werden. Als technische Reaktoren kommen Rohrbündel, Schachtöfen, Hordenreaktoren oder Wirbelbettreaktoren in Frage. Rohrbündel haben sich als Reaktoren für das erfindungsgemäße Verfahren besonders bewährt. Reaktionsrohre hierfür haben Durchmesser von 20 bis 40 mm und Längen von 1 bis 6 m. An den so angeordneten Katalysator werden dampfförmiges Benzol und die verdampfte Carbonsäure, gegebenenfalls in einem leichtem Inertgasstrom, herangeleitet. Eine besonders bevorzugte Temperatur ist hierbei der Bereich von 250-280°C. Als Inertgas kommen Stickstoff, Edelgase und andere in Frage. Das die Katalysatorzone verlassende Gemisch wird kondensiert und in an sich bekannter Weise aufgearbeitet.

Bei der Reaktion des erfindungsgemäßen Verfahrens entstehen sehr wenig, in vielen Fällen praktisch keine kondensierbaren flüchtigen Nebenprodukte. Bei Temperaturen am oberen Rand des beschriebenen Bereiches bildet sich jedoch etwas CO₂, das vermutlich durch Decarboxylierung der Carbonsäure entsteht. Es ist daher vorteilhaft, bei einer Temperatur im mittleren oder unteren Teil des angegebenen Bereiches zu arbeiten und hierbei einen nur partiellen Umsatz der Carbonsäure in Kauf zu nehmen. Im allgemeinen wählt man die Bedingungen so, daß 5 - 50 % der eingesetzten Carbonsäure umgesetzt werden.

So wird beispielsweise beim Umsatz von Benzol mit Benzoesäure bei einem Benzoesäure-Umsatz von 20-40 % der eingesetzten Benzoesäure gearbeitet.

Bei nachlassender Reaktivität kann der Katalysator nach bekannten Methoden reaktiviert werden, etwa durch Abbrennen mit Luft oder anderen Sauerstoff enthaltenden Gasgemischen.

Zur Aufarbeitung kann beispielsweise der gesamte Produktstrom kondensiert werden, woraus die Benzoesäure kristallisiert wird. Das Kristallisat kann sodann mechanisch abgetrennt werden, und die Mutterlauge wird vom überschüssigen Benzol destillativ befreit. Als Rückstand erhält man ein rohes Acylbenzol, das durch Destillation oder Kristallisation weiter gereinigt werden kann. Man kann jedoch auch den Produktstrom partiell kondensieren und auf diese Weise fraktioniert das Benzol, die nicht umgesetzte Carbonsäure und das Acylbenzol trennen. In vielen Fällen ist eine integrale Kondensation des Produktstroms und eine anschließende Fraktionierung geeignet. Nicht umgesetztes Benzol und nicht umgesetzte Carbonsäure können in bekannter Weise in die Reaktion zurückgeführt werden.

## Patentansprüche

1. Verfahren zur Herstellung von Acylbenzolen der Formel 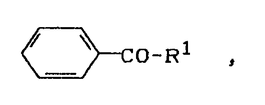 worin
R¹ für -C₆H₄-R², worin R² Wasserstoff, Methyl, Ethyl, Isopropyl oder geradkettiges c₃-c₁₄-Alkyl bedeutet, steht,
durch Umsetzung von Benzol mit Carbonsäuren an sauren Katalysatoren, dadurch gekennzeichnet, daß man eine Carbonsäure der Formel
HOOC-R¹ ,
worin R¹ die obige Bedeutung hat,
in freier Form mit Benzol im Molverhältnis Benzol: Carbonsäure = 1-50:1 bei einer Temperatur von 200-400°C in der Gasphase umsetzt und als sauren Katalysator einen Zeolith vom Pentasil-Typ mit einem SiO₂/AlO₂-Verhältnis von 15-500:1 benutzt, wobei als austauschbare Kationen solche von Mg oder Ca vorliegen.

2. Verfahren nach Anspruch 1, dadurch gekennzeichnet, daß das Molverhältnis Benzol:Carbonsäure 5-30:1 beträgt.

3. Verfahren nach Anspruch 2, dadurch gekennzeichnet, daß das Molverhältnis Benzol:Carbonsäure 10-20:1 beträgt.

4. Verfahren nach Anspruch 1, dadurch gekennzeichnet, daß Benzol und Benzoesäure zu Benzophenon umgesetzt werden.

5. Verfahren nach Anspruch 4, dadurch gekennzeichnet, daß die Umsetzung bei 250-300°C bis zu einem Benzoesäure-Umsatz von 20-40 % der eingesetzten Benzoesäure durchgeführt wird.

6. Verfahren nach Anspruch 1, dadurch gekennzeichnet, daß das SiO₂/AlO₂-Verhältnis 30-300:1 beträgt.

7. Verfahren nach Anspruch 6, dadurch gekennzeichnet, daß das SiO₂/AlO₂-Verhältnis 50-150:1 beträgt.

## Claims

1. Process for the preparation of acylbenzenes of the formula 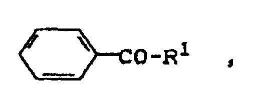 wherein
R¹ represents -C₆H₄-R², wherein R² denotes hydrogen, methyl, ethyl, isopropyl or straight-chain C₃-C₁₄-alkyl,
by reaction of benzene with carboxylic acids over acid catalysts, characterized in that a carboxylic acid of the formula
HOOC-R¹ ,
wherein R¹ has the above meaning,
is reacted in the free form with benzene in a molar ratio of benzene:carboxylic acid = 1-50:1 at a temperature of 200-400°C in the gas phase and a zeolite of the pentasil type having an SiO₂/AlO₂ ratio of 15-500:1 is used as the acid catalyst, cations of Mg or Ca being present as exchangeable cations.

2. Process according to Claim 1, characterized in that the molar ratio of benzene:carboxylic acid is 5-30:1.

3. Process according to Claim 2, characterized in that the molar ratio of benzene:carboxylic acid is 10-20:1.

4. Process according to Claim 1, characterized in that benzene and benzoic acid are reacted to give benzophenone.

5. Process according to Claim 4, characterized in that the reaction is carried out at 250-300°C up to a benzoic acid conversion of 20-40% of the benzoic acid employed.

6. Process according to Claim 1, characterized in that the SiO₂/AlO₂ ratio is 30-300:1.

7. Process according to Claim 6, characterized in that the SiO₂/AlO₂ ratio is 50-150:1.

## Revendications

1. Procédé de préparation d'acylbenzènes de formule 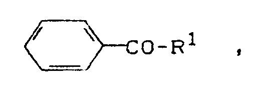 dans laquelle
R¹ représente -C₆H₄-R², dans laquelle R² est l'hydrogène, le groupement méthyle, le groupement éthyle, le groupement isopropyle ou un groupement alkyle en C₃-C₁₄ à chaîne linéaire,
par réaction de benzène avec des acides carboxyliques sur des catalyseurs acides, caractérisé en ce qu'on fait réagir un acide carboxylique de formule
HOOC-R¹
dans laquelle R¹ a la signification ci-dessus,
sous forme libre avec du benzène en un rapport molaire du benzène à l'acide carboxylique de 1-50 : 1, à une température comprise entre 200 et 400°C en phase gazeuse, et en ce qu'on utilise en tant que catalyseur acide une zéolite du type pentasile ayant un rapport SiO₂/AlO₂ de 15-500 : 1, étant entendu que des cations tels que Mg ou Ca sont présents en tant que cations échangeables.

2. Procédé selon la revendication 1, caractérisé en ce que le rapport molaire du benzène à l'acide carboxylique vaut 5-30 : 1.

3. Procédé selon la revendication 2, caractérisé en ce que le rapport molaire du benzène à l'acide carboxylique vaut 10-20 : 1.

4. Procédé selon la revendication 1, caractérisé en ce qu'on transforme le benzène et l'acide benzoïque en benzophénone.

5. Procédé selon la revendication 4, caractérisé en ce qu'on met en oeuvre la réaction entre 250 et 300°C jusqu'à obtenir une réaction de 20 à 40 % de l'acide benzoïque utilisé.

6. Procédé selon la revendication 1, caractérisé en ce que le rapport SiO₂/AlO₂ vaut 30-300 : 1.

7. Procédé selon la revendication 6, caractérisé en ce que le rapport du type SiO₂/AlO₂ vaut 50-150 : 1.
